# EUROPÄISCHE PATENTSCHRIFT

(11) **EP 1 401 272 B1**
(45) Veröffentlichungstag und Bekanntmachung des Hinweises auf die Patenterteilung: **05.07.2006**
(21) Anmeldenummer: 02743170.9
(22) Anmeldetag: 10.06.2002
(51) Int. Cl.: A01N 25/04, A01N 25/30

(54) **SUSPENSIONSKONZENTRATE AUF ÖLBASIS**
OIL-BASED SUSPENSION CONCENTRATES
SUSPENSIONS CONCENTREES A BASE D'HUILE

(30) Priorität: 21.06.2001 DE 10129855
(43) Veröffentlichungstag der Anmeldung: 31.03.2004
(60) Teilanmeldung: 06001909.8
(73) Patentinhaber: Bayer CropScience AG, 40789 Monheim (DE)
(72) Erfinder: VERMEER, Ronald, 51371 Leverkusen (DE); BAUR, Peter, 65817 Eppstein (DE); ROSENFELDT, Frank, 40764 Langenfeld (DE)
(86) Internationale Anmeldenummer: PCT/EP2002/006323
(87) Internationale Veröffentlichungsnummer: WO 2003/000053

(56) Entgegenhaltungen:
- EP-A- 0 313 317
- EP-A- 0 456 198
- EP-A- 0 789 999
- US-A- 6 165 940
- DATABASE WPI Derwent Publications Ltd., London, GB; AN 1992-076724 XP002212964 HODOGAYA CHEM IND CO LTD: "Oily herbicidal suspension for application to water surfaces- includes benzanilide and pyrazole carboxylate, mineral or plant oils and surfactants" & JP 03 020996 A 29. Januar 1991 (1991-01-29)

## Beschreibung

Die vorliegende Erfindung betrifft neue, ölbasierte Suspensionskonzentrate von agrochemischen Wirkstoffen, ein Verfahren zur Herstellung dieser Formulierungen und deren Verwendung zur Applikation der enthaltenen Wirkstoffe.

Es sind bereits zahlreiche wasserfreie Suspensionskonzentrate von agrochemischen Wirkstoffen bekannt geworden. So werden in der EP-A 0 789 999 Formulierungen dieses Typs beschrieben, die neben Wirkstoff und Öl ein Gemisch verschiedener Tenside, - darunter auch solche, die als Penetrationsförderer dienen -, sowie ein hydrophobiertes Alumoschichtsilikat als Verdickungsmittel enthalten. Die Stabilität dieser Zubereitungen ist gut. Nachteilig ist aber, dass zwingend ein Verdickungsmittel vorhanden ist, denn dadurch wird die Herstellung aufwendiger. Außerdem absorbiert das Verdickungsmittel jeweils einen Teil der zugesetzten Menge an Penetrationsförderer, der deshalb für seine eigentliche Funktion nicht zur Verfügung steht.

Weiterhin sind aus der US-A 6 165 940 schon nicht-wässrige Suspensionskonzentrate bekannt, in denen außer agrochemischern Wirkstoff, Penetrationsförderer und Tensid bzw. Tensid-Gemisch ein organisches Solvens vorhanden ist, wobei als derartige Lösungsmittel auch Paraffinöl oder Pflanzenöl-Ester in Frage kommen. Die biologische. Wirksamkeit und die Stabilität der aus diesen Formulierungen durch Verdünnen mit Wasser herstellbaren Spritzbrühen ist jedoch nicht immer ausreichend.

Es wurden nun neue Suspensionskonzentrate auf Ölbasis gefunden, die aus
- mindestens einem bei Raumtemperatur festen agrochemischen Wirkstoff,
- mindestens einem Penetrationsförderer,
- mindestens einem Pflanzenöl,
- mindestens einem nicht-ionischen Tensid bzw. Dispergierhilfsmittel und/oder mindestens einem anionischen Tensid bzw. Dispergierhilfsmittel und
- gegebenenfalls einem oder mehreren Zusatzstoffen aus den Gruppen der Emulgiermittel, der schaumhemmenden Mittel, der Konservierungsmittel, der Antioxydantien, und/oder der Farbstoffe
bestehen.

Weiterhin wurde gefunden, dass sich die erfindungsgemäßen Suspensionkonzentrate auf Ölbasis herstellen lassen, indem man
- mindestens einen bei Raumtemperatur festen agrochemischen Wirkstoff,
- mindestens einen Penetrationsförderer,
- mindestens ein Pflanzenöl,
- mindestens ein nicht-ionisches Tensid bzw. Dispergierhilfsmittel und/oder mindestens ein anionisches Tensid bzw. Dispergierhilfsmittel und
- gegebenenfalls einen oder mehrere Zusatzstoffe aus den Gruppen der Emulgiermittel, der schaumhemmenden Mittel, der Konservierungsmittel, der Antioxydantien, und/oder der Farbstoffe
miteinander vermischt und die entstehende Suspension gegebenenfalls anschließend mahlt.

Schließlich wurde gefunden, dass sich die erfindungsgemäßen Suspensionskonzentrate auf Ölbasis sehr gut zur Applikation der enthaltenen agrochemischen Wirkstoffe auf Pflanzen und/oder deren Lebensraum eignen.

Es ist als äußerst überraschend zu bezeichnen, dass die erfindungsgemäßen Suspensionskonzentrate auf Ölbasis eine sehr gute Stabilität aufweisen, obwohl sie kein Verdickungsmittel enthalten. Unerwartet ist auch, dass sie eine deutlich bessere biologische Wirksamkeit zeigen als die am ähnlichsten zusammengesetzten, vorbekannten Formulierungen. Im Übrigen übertreffen die erfindungsgemäßen ölbasierten Suspensionskonzentrate hinsichtlich ihrer Aktivität überraschenderweise auch analoge Zubereitungen, die neben den anderen Komponenten entweder nur Penetrationsförderer oder nur Pflanzenöl enthalten. Ein solcher synergistischer Effekt war aufgrund des vorbeschriebenen Standes der Technik nicht vorhersehbar.

Die erfindungsgemäßen Suspensionskonzentrate auf Ölbasis zeichnen sich auch durch eine Reihe von Vorteilen aus. So ist deren Herstellung weniger aufwendig als die Zubereitung entsprechender Formulierungen, in denen Verdickungsmittel vorhanden sind. Vorteilhaft ist weiterhin, dass beim Verdünnen der erfindungsgemäßen Konzentrate mit Wasser weder eine signifikante Aufrahmung noch eine störende Flockenbildung eintritt, was bei entsprechenden vorbekannten Zubereitungen häufig der Fall ist. Schließlich begünstigen die erfindungsgemäßen Formulierungen die biologische Wirksamkeit der enthaltenen aktiven Komponenten, so dass im Vergleich zu herkömmlichen Zubereitungen entweder eine höhere Wirksamkeit erzielt wird oder weniger Wirkstoff erforderlich ist.

Unter festen, agrochemischen Wirkstoffen sind im vorliegenden Zusammenhang alle zur Pflanzenbehandlung üblichen Substanzen zu verstehen, deren Schmelzpunkt oberhalb von 20°C liegt. Vorzugsweise genannt seien Fungizide, Bakterizide, Insektizide, Akarizide, Nematizide, Molluskizide, Herbizide, Pflanzenwuchsregulatoren, Pflanzennährstoffe und Repellents.

Als Beispiele für Fungizide seien genannt:
2-Anilino-4-methyl-6-cyclopropyl-pyrimidin; 2',6'-Dibromo-2-methyl-4'-trifluoromethoxy-4'-trifluoromethyl-1,3-thiazol-5-carboxanilid; 2,6-Dichloro-N-(4-trifluoromethylbenzyl)-benzamid; (E)-2-Methoximino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl}-3-methoxyacrylat; Methyl-(E)-methoximino[alpha-(o-tolyloxy)-o-tolyl]-acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram, Carpropamid,
Dichlorophen, Diclobutrazol, Dichlofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox, Fenhexamid,
Guazatine,
Hexachlorobenzol, Hexaconazol, Hymexazol,
Imazalil, Imibenconazol, Iminoctadin, Iprobenfos (IBP), Iprodion, Isoprothiolan, Iprovalicarb,
Kasugamycin, Kupfer-Zubereitungen, wie: Kupferhydroxid, Kupfemaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickeldimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB), Quinoxyfen,
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol, Trifloxystrobin,
Validamycin A, Vinclozolin,
Zineb, Ziram und
2-[2-(1-Chlor-cyclopropyl)-3-(2-chlorphenyl)-2-hydroxypropyl]-2,4-dihydro-[1,2,4]-triazol-3-thion.

Als Beispiele für Bakterizide seien genannt:
Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

Als Beispiele für Insektizide, Akarizide und Nematizide seien genannt:
Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, 4-Bromo-2-(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chloethocarb, Chloretoxyfos, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton-M, Demeton-S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazuron, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram,
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimiphos, Teflubenzuron, Tefluthrin, Temephos, Terbam, Terbufos, Tetrachlorvinphos, Thiacloprid, Thiafenox, Thiamethoxam, Thiodicarb, Thiofanox, Thiomethon, Thionazin, Thuringiensin, Tralomethrin, Transfluthrin, Triarathen, Triazophos, Triazuron, Trichlorfon, Triflumuron, Trimethacarb,
Vamidothion, XMC, Xylylcarb, Zetainethrin.

Als Beispiele für Molluskizide seien Metaldehyd und Methiocarb genannt.

Als Beispiele für Herbizide seien genannt:
Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfopmethyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimumn-ethyl, Chlorsulfuron, Cinosulfuron, Metsulfuron-methyl, Nicosulfiuon, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluomchloridone, Glufosinate, Glyphosate, Isoxaben, Pyridate, Quinchlorac, Quinmerac, Sulphosate und Tridiphane. Desweiteren seien 4-Amino-N-(1,1-dimethylethyl)-4,5-dihydro-3-(1-metylethyl)-5-oxo-1H-1,2,4-triazole-1-carboxamide und Benzoesäure-2-((((4,5-dihdydro-4-methyl-5-oxo-3-propoxy-1H-1,2,4-triazol-1-yl)carbonyl)amino)sulfonyl)-methylester genannt.

Als Beispiele für Pflanzenwuchsregulatoren seien Chlorcholinchlorid und Ethephon genannt.

Als Beispiele für Pflanzennährstoffe seien übliche anorganische oder organische Dünger zur Versorgung von Pflanzen mit Makro- und/oder Mikronährstoffen genannt.

Als Beispiele für Repellents seien Diethyl-tolylamid, Ethylhexandiol und Butopyronoxyl genannt.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Bevorzugt sind Alkanolalkoxylate der Formel

R-O-(-AO)ₘH (I)

in welcher
- R: für geradkettiges oder verzweigtes Alkyl mit 4 bis 20 Kohlenstoffatomen steht,
- AO: für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
- m: für Zahlen von 2 bis 30 steht.

Eine besonders bevorzugte Gruppe von Penetrationsförderern sind Alkanolalkoxylate der Formel

R-O-(-EO-)ₙ-H (Ia)

in welcher
- R: die oben angegebene Bedeutung hat,
- EO: für -CH₂-CH₂-O- steht und
- n: für Zahlen von 2 bis 20 steht.

Eine weitere besonders bevorzugte Gruppe von Penetrationsförderern sind Alkanolalkoxylate der Formel

R-O-(-EO-)ₚ-(-PO-)_{q}-H (Ib)

in welcher
- R: die oben angegebene Bedeutung hat,
- EO: für -CH₂-CH₂-O- steht,
- PO: für 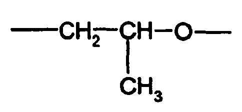 steht,
- p: für Zahlen von 1 bis 10 steht und
- q: für Zahlen von 1 bis 10 steht.

Eine weitere besonders bevorzugte Gruppe von Penetradonsförderern sind Alkanol-Alkoxylate der Formel

R-O-(-PO-)ᵣ-(EO-)ₛ-H (Ic)

in welcher
- R: die oben angegebene Bedeutung hat,
- EO: für -CH₂-CH₂-O- steht,
- PO: für 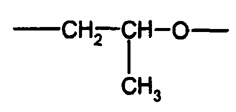 steht,
- r: für Zahlen von 1 bis 10 steht und
- s: für Zahlen von 1 bis 10 steht.

Eine weitere besonders bevorzugte Gruppe von Penetrationsförderern sind Alkanol-Alkoxylate der Formel

CH₃-(CH₂)ₜ-CH₂-O-(-CH₂-CH₂-O-)ᵤ- H (Id)

in welcher
- t: für Zahlen von 8 bis 13 steht
und
- u: für Zahlen von 6 bis 17 steht.

In den zuvor angegebenen Formeln steht
- R: vorzugsweise für Butyl, i-Butyl, n-Pentyl, i-Pentyl, Neopentyl, n-Hexyl, i-Hexyl, n-Octyl, i-Octyl, 2-Ethyl-hexyl, Nonyl, i-Nonyl, Decyl, n-Dodecyl, i-Dodecyl, Lauryl, Myristyl, i-Tridecyl, Trimethyl-nonyl, Palmityl, Stearyl oder Eicosyl.

Als Beispiel für ein Alkanol-Alkoxylat der Formel (Ic) sei 2-Ethyl-hexyl-alkoxylat der Formel 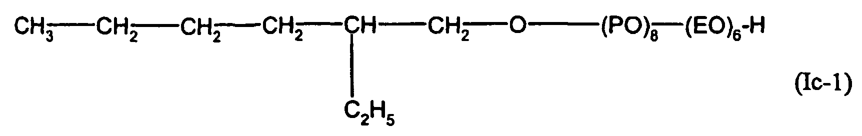 in welcher
- EO: für -CH₂-CH₂-O- steht,
- PO: für 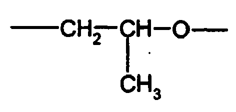 steht und
die Zahlen 8 und 6 Durchschnittswerte darstellen.

Besonders bevorzugte Alkanol-Alkoxylate der Formel (Id) sind Verbindungen dieser Formel, in denen
- t: für Zahlen von 9 bis 12 steht und
- u: für Zahlen von 7 bis 9 steht.

Die Alkanol-Alkoxylate sind durch die obigen Formeln allgemein definiert. Bei diesen Substanzen handelt es sich um Gemische von Stoffen des angegebenen Typs mit unterschiedlichen Kettenlängen. Für die Indices errechnen sich deshalb Durchschnittswerte, die auch von ganzen Zahlen abweichen können.

Beispielhaft genannt sei Alkanol-Alkoxylat der Formel (Id), in welcher
- t: für den Durchschnittswert 10,5 steht und
- u: für den Durchschnittswert 8,4 steht.

Die Alkanol-Alkoxylate der angegebenen Formeln sind bekannt oder lassen sich nach bekannten Methoden herstellen (vgl. WO 98-35 553, WO 00-35 278 und EP-A 0 681 865).

Als Pflanzenöle kommen alle üblicherweise in agrochemischen Mitteln einsetzbaren, aus Pflanzen gewinnbaren Öle in Frage. Beispielhaft genannt seien Sonnenblumenöl, Rapsöl, Olivenöl, Rizinusöl, Rüböl, Maiskernöl, Baumwollsaatöl und Sojabohnenöl.

Die erfindungsgemäßen Suspensionskonzentrate auf Ölbasis enthalten mindestens ein nicht-ionisches Tensid bzw. Dispergierhilfsmittel und/oder mindestens ein anionisches Tensid bzw. Dispergierhilfsmittel.

Als nicht-ionische Tenside bzw. Dispergierhilfsmittel kommen alle üblicherweise in agrochemischen Mitteln einsetzbaren Stoffe dieses Typs in Betracht. Vorzugsweise genannt seien Polyethylenoxid-polypropylenoxid-Blockcopolymere, Polyethylenglykolether von linearen Alkoholen, Umsetzungsprodukte von Fettsäuren mit Ethylenoxid und/oder Propylenoxid, ferner Polyvinylalkohol, Polyvinylpyrrolidon, Mischpolymerisate aus Polyvinylalkohol und Polyvinylpyrrolidon sowie Copolymerisate aus (Meth)acrylsäure und (Meth)acrylsäureestem, weiterhin Alkylethoxylate und Alkylarylethoxylate, die gegebenenfalls phosphatiert und gegebenenfalls mit Basen neutralisiert sein können, wobei Sorbitolethoxylate beispielhaft genannt seien, sowie Polyoxyalkylenamin-Derivate.

Als anionische Tenside kommen alle üblicherweise in agrochemischen Mitteln einsetzbaren Substanzen dieses Typs in Frage. Bevorzugt sind Alkalimetall- und Erdalkalimetall-Salze von Alkylsulfonsäuren oder Alkylarylsulfonsäuren.

Eine weitere bevorzugte Gruppe von anionischen Tensiden bzw. Dispergierhilfsmitteln sind in Pflanzenöl wenig lösliche Salze von Polystyrolsulfonsäuren, Salze von Polyvinylsulfonsäuren, Salze von Naphthalinsulfonsäure-Formaldehyd-Kondensationsprodukten, Salze von Kondensationsprodukten aus Naphthalinsulfonsäure, Phenolsulfonsäure und Formaldehyd sowie Salze von Ligninsulfonsäure.

Als Zusatzstoffe, die in den erfindungsgemäßen Formulierungen enthalten sein können, kommen Emulgatoren, schaumhemmende Mittel, Konservierungsmittel, Antioxydantien und Farbstoffe in Betracht.

Bevorzugte Emulgatoren sind ethoxylierte Nonylphenole, Umsetzungsprodukte von Alkylphenolen mit Ethylenoxid und/oder Propylenoxid, ethoxylierte Arylalkylphenole, weiterhin ethoxylierte und propoxylierte Arylalkylphenole, sowie sulfatierte oder phosphatierte Arylalkylethoxylate bzw. -ethoxy-propoxylate, wobei Sorbitan-Derivate, wie Polyethylenoxid-Sorbitan-Fettsäureester und Sorbitan-Fettsäureester, beispielhaft genannt seien.

Als schaumhemmende Stoffe kommen alle üblicherweise für diesen Zweck in agrochemischen Mitteln einsetzbaren Substanzen in Betracht. Bevorzugt sind Silikonöle und Magnesiumstearat.

Als Konservierungsmittel kommen alle üblicherweise für diesen Zweck in agrochemischen Mitteln dieses Typs einsetzbaren Substanzen in Frage. Als Beispiele genannt seien Preventol® (Fa. Bayer AG) und Proxel®.

Als Antioxydantien kommen alle üblicherweise für diesen Zweck in agrochemischen Mitteln einsetzbaren Substanzen in Betracht. Bevorzugt ist Butylhydroxytoluol.

Als Farbstoffe kommen alle üblicherweise für diesen Zweck in agrochemischen Mitteln einsetzbaren Substanzen in Frage. Beispielhaft genannt seien Titandioxid, Farbruß, Zinkoxid und Blaupigmente sowie Permanentrot FGR.

Der Gehalt an den einzelnen Komponenten kann in den erfindungsgemäßen Suspensionskonzentraten auf Ölbasis innerhalb eines größeren Bereiches variiert werden. So liegen die Konzentrationen
- an agrochemischen Wirkstoffen im allgemeinen zwischen 5 und 30 Gew.-%, vorzugsweise zwischen 10 und 25 Gew.-%,
- an Penetrationsförderer im allgemeinen zwischen 5 und 55 Gew.-%, vorzugsweise zwischen 15 und 40 Gew.-%,
- an Pflanzenöl im allgemeinen zwischen 15 und 55 Gew.-%, vorzugsweise zwischen 20 und 50 Gew.-%,
- an Tensiden bzw. Dispergierhilfsmitteln im allgemeinen zwischen 2,5 und 30 Gew.-%, vorzugsweise zwischen 5,0 und 25 Gew.-% und
- an Zusatzstoffen im allgemeinen zwischen 0 und 25 Gew.-%, vorzugsweise zwischen 0 und 20 Gew.-%.

Die Herstellung der erfindungsgemäßen Suspensionskonzentrate auf Ölbasis erfolgt in der Weise, dass man die Komponenten in den jeweils gewünschten Verhältnissen miteinander vermischt. Die Reihenfolge, in der die Bestandteile miteinander vermengt werden, ist beliebig. Zweckmäßigerweise setzt man die festen Komponenten in feingemahlenem Zustand ein. Es ist aber auch möglich, die nach dem Vermengen der Bestandteile entstehende Suspension zunächst einer Grob- und dann einer Feinmahlung zu unterziehen, so dass die mittlere Teilchengröße unterhalb von 20 µm liegt. Bevorzugt sind Suspensionskonzentrate, in denen die festen Partikel eine mittlere Teilchengröße zwischen 1 und 10 µm aufweisen.

Die Temperaturen können bei der Durchführung des erfindungsgemäßen Verfahrens in einem bestimmten Bereich variiert werden. Man arbeitet im allgemeinen bei Temperaturen zwischen 10°C und 60°C, vorzugsweise zwischen 15°C und 40°C.

Zur Durchführung des erfindungsgemäßen Verfahrens kommen übliche Misch- und Mahlgeräte in Betracht, die zur Herstellung von agrochemischen Formulierungen eingesetzt werden.

Bei den erfindungsgemäßen Suspensionskonzentraten auf Ölbasis handelt es sich um Formulierungen, die auch nach längerer Lagerung bei erhöhten Temperaturen oder in der Kälte stabil bleiben, da kein Kristallwachstum beobachtet wird. Sie lassen sich durch Verdünnen mit Wasser in homogene Spritzflüssigkeiten überführen. Die Anwendung dieser Spritzflüssigkeiten erfolgt nach üblichen Methoden, also zum Beispiel durch Verspritzen, Gießen oder Injizieren.

Die Aufwandmenge an den erfindungsgemäßen Suspensionskonzentraten auf Ölbasis kann innerhalb eines größeren Bereiches variiert werden. Sie richtet sich nach den jeweiligen agrochemischen Wirkstoffen und nach deren Gehalt in den Formulierungen.

Mit Hilfe der erfindungsgemäßen Suspensionskonzentrate auf Ölbasis lassen sich agrochemische Wirkstoffe in besonders vorteilhafter Weise auf Pflanzen und/oder deren Lebensraum ausbringen. Die enthaltenen agrochemischen Wirkstoffe entfalten dabei eine bessere biologische Wirksamkeit als bei Applikation in Form der entsprechenden herkömmlichen Formulierungen.

Die Erfindung wird durch die folgenden Beispiele veranschaulicht.

### Herstellungsbeispiele

### Beispiel 1

Zur Herstellung eines Suspensionskonzentrates werden

| | |
|---|---|
| 48,4 g | Thiacloprid |
| 45,6 g | eines Gemisches aus Alkylarylsulfonat, Ethylhexanol und Alkanolethoxylat |
| 40,0 g | Polyoxyethylen-sorbitol-oleat, |
| 0,4 g | Silikonöl und |
| 0,8 g | Butylhydroxytoluol |

unter Rühren bei Raumtemperatur in ein Gemisch aus
88,0 g 2-Ethyl-hexyl-alkoxylat der Formel 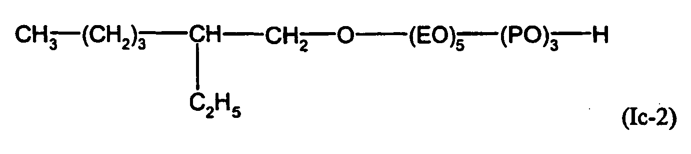 in welcher
EO für -CH₂-CH₂-O- seht,
PO für 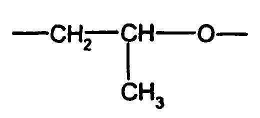 steht und
die Zahlen 5 und 3 Durchschnittswerte darstellen,
und
176,8 g Sonnenblumenöl
gegeben. Nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90% der Feststoffpartikel eine Teilchengröße unter 6 µm aufweisen.

### Beispiel 2

Zur Herstellung eines Suspensionskonzentrates werden

| | |
|---|---|
| 78,2 g | Thiacloprid |
| 40,0 g | eines Gemisches aus Calciumalkylarylsulfonat, Alkylphenolethoxylat und Naphtha-Lösung |
| 40,0 g | Polyoxyethylen-sorbitol-oleat |
| 0,4 g | Silikonöl und |
| 0,8 g | Butylhydroxytoluol |

unter Rühren bei Raumtemperatur in ein Gemisch aus

| | |
|---|---|
| 80,0 g | 2-Ethyl-hexyl-alkoxylat der Formel (Ic-2) und |
| 160,6 g | Sonnenblumenöl |

gegeben. Nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90% der Feststoffpartikel eine Teilchengröße unter 6 µm aufweisen.

### Beispiel 3

Zur Herstellung eines Suspensionskonzentrates werden

| | |
|---|---|
| 50,4 g | Thiacloprid |
| 27,5 g | eines Gemisches aus Alkylarylsulfonat und Ethylhexanol |
| 5,25 g | einfach verzweigtes Alkanolethoxylat mit durchschnittlich 15 Ethylenoxid-Gruppen |
| 25,0 g | Polyoxyethylen-sorbitol-oleat |
| 0,25 g | Silikonöl und |
| 0,5 g | Butylhydroxytoluol |

unter Rühren bei Raumtemperatur in ein Gemisch aus

| | |
|---|---|
| 50,0 g | 2-Ethyl-hexyl-alkoxylat der Formel (Ic-2) und |
| 91,1 g | Sonnenblumenöl |

gegeben. Nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90% der Feststoffpartikel eine Teilchengröße unter 6 µm aufweisen.

### Beispiel 4

Zur Herstellung eines Suspensionskonzentrates werden

| | |
|---|---|
| 49,4 g | Thiacloprid |
| 23,75 g | eines Gemisches aus Alkylarylsulfonat und Ethylhexanol |
| 4,5 g | einfach verzweigtes Alkanolethoxylat mit durchschnittlich 15 Ethylenoxid-Gruppen |
| 25,0 g | Polyoxyethylen-sorbitol-oleat |
| 0,25 g | Silikonöl und |
| 0,5 g | Butylhydroxytoluol |

unter Rühren bei Raumtemperatur in ein Gemisch aus

| | |
|---|---|
| 50,0 g | 2-Ethyl-hexyl-alkoxylat der Formel (Ic-2) und |
| 96,6 g | Sonnenblumenöl |

gegeben. Nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90% der Feststoffpartikel eine Teilchengröße unter 6 µm aufweisen.

### Beispiel 5

Zur Herstellung eines Suspensionskonzentrates werden

| | |
|---|---|
| 692,54 g | Thiacloprid |
| 300,0 g | eines Gemisches aus Alkylarylsulfonat, Alkanolethoxylat und Naphtha-Lösung |
| 300,0 g | Polyoxyethylen -sorbitol-oleat |
| 3,0 g | Silikonöl und |
| 6,0 g | Butylhydroxytoluol |

unter Rühren bei Raumtemperatur in ein Gemisch aus

| | |
|---|---|
| 600,0 g | 2-Ethyl-hexyl-alkoxylat der Formel (Ic-2) und |
| 1098,46 g | Sonnenblumenöl |

gegeben. Nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90% der Feststoffpartikel eine Teilchengröße unter 6 µm aufweisen.

### Beispiel 6

Zur Herstellung eines Suspensionskonzentrates werden

| | |
|---|---|
| 577,1 g | Thiacloprid |
| 327,5 g | eines Gemisches aus Alkylaryl-sulfonat, Ethylhexanol und Alkanolethoxylat |
| 250,0 g | Polyoxyethylen-sorbitol-oleat |
| 2,5 g | Silikonöl und |
| 5,0 g | Butylhydroxytoluol |

unter Rühren bei Raumtemperatur in ein Gemisch aus

| | |
|---|---|
| 500,0 g | 2-Ethyl-hexyl-alkoxylat der Formel (Ic-2) und |
| 837,9 g | Sonnenblumenöl |

gegeben. Nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90% der Feststoffpartikel eine Teilchengröße unter 6 µm aufweisen.

### Beispiel 7

Zur Herstellung eines Suspensionskonzentrates werden

| | |
|---|---|
| 44,4 g | Thiacloprid |
| 5,6 g | β-Cyfluthrin |
| 49,7 g | eines Gemisches aus Alkylarylsulfonat, Ethylhexanol und Alkanolethoxylat |
| 44,0 g | Polyoxyethylen-sorbitol-oleat |
| 0,4 g | Silikonöl und |
| 0,8 g | Butylhydroxytoluol |

unter Rühren bei Raumtemperatur in ein Gemisch aus

| | |
|---|---|
| 101,3 g | 2-Ethyl-hexyl-alkoxylat der Formel (Ic-2) und |
| 193,8 g | Sonnenblumenöl |

gegeben. Nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90% der Feststoffpartikel eine Teilchengröße unter 6 µm aufweisen.

### Beispiel 8

Zur Herstellung eines Suspensionskonzentrates werden

| | |
|---|---|
| 121,0 g | Thiacloprid |
| 15,2 g | β-Cycluthrin |
| 78,6 g | eines Gemisches aus Alkylarylsulfonat, Ethylhexanol und Alkanolethoxylat |
| 60,0 g | Polyoxyethylen-sorbitol-oleat |
| 0,6 g | Silikonöl und |
| 1,2 g | Butylhydroxytoluol |

unter Rühren bei Raumtemperatur in ein Gemisch aus

| | |
|---|---|
| 120,0 g | 2-Ethyl-hexyl-alkoxylat der Formel (Ic-2) und |
| 203,4 g | Sonnenblumenöl |

gegeben. Nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90% der Feststoffpartikel eine Teilchengröße unter 6 µm aufweisen.

### Beispiel 9

Zur Herstellung eines Suspensionskonzentrates werden

| | |
|---|---|
| 138,5 g | Thiacloprid, |
| 60,0 g | Polyoxyethylen-sorbitol-oleat |
| 12,0 g | Polystyrol-Acrylsäure-Copolymer |
| 48,0 g | Polyoxyethylen-Fettsäureglycerid |
| 0,6 g | Silikonöl und |
| 1,2 g | Butylhydroxytoluol |

unter Rühren bei Raumtemperatur in ein Gemisch aus
- 120,0 g: Alkanol-Alkoxylat der Formel

R-O-(EO)₃-(PO)₆-H

in welcher
R für Alkyl mit 12 bis 14 Kohlenstoffatomen steht,
EO für -CH₂-CH₂-O steht,
PO für 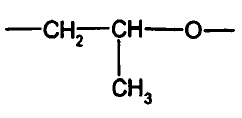 steht und
die Zahlen 3 und 6 Durchschnittswerte darstellen, und
- 219,7 g: Rapsöl
gegeben. Nach beendeter Zugabe wird noch 10 Minuten bei Raumtemperatur nachgerührt. Die dabei entstehende homogene Suspension wird zunächst einer Grob- und dann einer Feinmahlung unterworfen, so dass eine Suspension erhalten wird, in der 90% der Feststoffpartikel eine Teilchengröße unter 6 µm aufweisen.

### Verwendungsbeispiele

### Beispiel I

### Stabilitätstest

Zur Bestimmung der Stabilität werden jeweils 100 g eines Suspensionskonzentrates der im Beispiel 2 beschriebenen Zusammensetzung über mehrere Wochen bei
-10°C,
Raumtemperatur,
+ 30°C,
+ 40°C
+ 54°C
wechselnden Temperaturen (6 Stunden bei -15°C, dann 6 Stunden bei +30°C)
gelagert. Die Versuchsergebnisse sind in den folgenden Tabellen zusammengestellt.

**Tabelle Ia Lagerung bei-10°C**

| | nach | | | | |
|---|---|---|---|---|---|
| | 2 Wochen | 4 Wochen | 8 Wochen | 16 Wochen | 26 Wochen |
| Sedimentvolumen in % ^{*)} | | | | | 99 |
| Bodensatz | | | | | keiner |
| Redispergierbarkeit | | | | | gut |
| Komgröße ^{**)} in µm | | | | | 5,35 |
| Gehalt an Wirkstoff in % | | | | | 19,8 |

| | | | | | |
|---|---|---|---|---|---|
| ^{*)} Sedimentvolumen = Volumen der Sediment-Phase im Verhältnis zum Gesamtvolumen der Probe. | | | | | |
| ^{**)} Gemessen wurde die mittlere Korngröße, die 90% der Feststoffpartikel in der Ölphase aufweisen. | | | | | |

**Tabelle Ib Lagerung bei Raumtemperatur**

| | nach | | | | |
|---|---|---|---|---|---|
| | 2 Wochen | 4 Wochen | 8 Wochen | 16 Wochen | 26 Wochen |
| Sedimentvolumen in % ^{*)} | | | 97 | | 89 |
| Bodensatz | | | keiner | | keiner |
| Redispergierbarkeit | | | gut | | gut |
| Korngröße ^{**)} in µm | | | 5,31 | | 5,86 |
| Gehalt an Wirkstoff in % | | | 20,1 | | 19,6 |

| | | | | | |
|---|---|---|---|---|---|
| ^{*)} Sedimentvolumen = Volumen der Sediment-Phase im Verhältnis zum Gesamtvolumen der Probe. | | | | | |
| ^{**)} Gemessen wurde die mittlere Korngröße, die 90% der Feststoffpartikel in der Ölphase aufweisen. | | | | | |

**Tabelle Ic Lagerung bei +30°C**

| | nach | | | | |
|---|---|---|---|---|---|
| | 2 Wochen | 4 Wochen | 8 Wochen | 16 Wochen | 26 Wochen |
| Sedimentvolumen in % ^{*)} | | | 94 | | 84 |
| Bodensatz | | | keiner | | keiner |
| Redispergierbarkeit | | | gut | | gut |
| Korngröße ^{**)} in µm | | | 6,57 | | 5,74 |
| Gehalt an Wirkstoff in % | | | 20,0 | | 19,8 |

| | | | | | |
|---|---|---|---|---|---|
| ^{*)} Sedimentvolumen = Volumen der Sediment-Phase im Verhältnis zum Gesamtvolumen der Probe. | | | | | |
| ^{**)} Gemessen wurde die mittlere KorngröBe, die 90% der Feststoffpartikel in der Ölphase aufweisen. | | | | | |

**Tabelle Id Lagerung bei +40°C**

| | nach | | | | |
|---|---|---|---|---|---|
| | 2 Wochen | 4 Wochen | 8 Wochen | 16 Wochen | 26 Wochen |
| Sedimentvolumen in % ^{*)} | | 93 | 92 | 87 | 82 |
| Bodensatz | | keiner | keiner | keiner | keiner |
| Redispergierbarkeit | | gut | gut | gut | gut |
| Komgröße ^{**)} in µm | | 6,01 | 6,29 | 7,08 | 6,4 |
| Gehalt an Wirkstoff in % | | 20,2 | 19,3 | 20,1 | 19,7 |

| | | | | | |
|---|---|---|---|---|---|
| ^{*)} Sedimentvolumen = Volumen der Sediment-Phase im Verhältnis zum Gesamtvolumen der Probe. | | | | | |
| ^{**)} Gemessen wurde die mittlere KorngröBe, die 90% der Feststoffpartikel in der Ölphase aufweisen. | | | | | |

**Tabelle Ie Lagerung bei +54°C**

| | nach | | | | |
|---|---|---|---|---|---|
| | 2 Wochen | 4 Wochen | 8 Wochen | 16 Wochen | 26 Wochen |
| Sedimentvolumen in % ^{*)} | 96 | 89 | 83 | | |
| Bodensatz | keiner | keiner | keiner | | |
| Redispergierbarkeit | gut | gut | gut | | |
| KorngröBe ^{**)} in µm | | 8,81 | 6,61 | | |
| Gehalt an Wirkstoff in % | 20,1 | 20,0 | 20,1 | | |

| | | | | | |
|---|---|---|---|---|---|
| ^{*)} Sedimentvolumen = Volumen der Sediment-Phase im Verhältnis zum Gesamtvolumen der Probe. | | | | | |
| ^{**)} Gemessen wurde die mittlere Korngröße, die 90% der Feststoffpartikel in der Ölphase aufweisen. | | | | | |

**Tabelle If Lagerung bei wechselnden Temperaturen**

| | nach | | | | |
|---|---|---|---|---|---|
| | 2 Wochen | 4 Wochen | 8 Wochen | 16 Wochen | 26 Wochen |
| Sedimentvolumen in % ^{*)} | | 98 | 99 | | |
| Bodensatz | | keiner | keiner | | |
| Redispergierbarkeit | | gut | gut | | |
| Korngröße ^{**)} in µm | | 5,62 | 6,17 | | |
| Gehalt an Wirkstoff in % | | 20,0 | 19,8 | | |

| | | | | | |
|---|---|---|---|---|---|
| ^{*)} Sedimentvolumen = Volumen der Sediment-Phase im Verhältnis zum Gesamtvolumen der Probe. | | | | | |
| ^{**)} Gemessen wurde die mittlere Korngröße, die 90% der Feststoffpartikel in der Ölphase aufweisen. | | | | | |

### Beispiel II

### Penetrationstest

In diesem Test wurde die Penetration von Wirkstoffen durch enzymatisch isolierte Kutikeln von Apfelbaumblättern gemessen.

Verwendet wurden Blätter, die in voll entwickeltem Zustand von Apfelbäumen der Sorte Golden Delicious abgeschnitten wurden. Die Isolierung der Kutikeln erfolgte in der Weise, dass
- zunächst auf der Unterseite mit Farbstoff markierte und ausgestanzte Blattscheiben mittels Vakuuminfiltration mit einer auf einen pH-Wert zwischen 3 und 4 gepufferten Pectinase-Lösung (0,2 bis 2 %ig) gefüllt wurden,
- dann Natriumazid hinzugefügt wurde und
- die so behandelten Blattscheiben bis zur Auflösung der ursprünglichen Blattstruktur und zur Ablösung der nicht zellulären Kutikula stehen gelassen wurden.

Danach wurden nur die von Spaltöffnungen und Haaren freien Kutikeln der Blattoberseiten weiter verwendet. Sie wurden mehrfach abwechselnd mit Wasser und einer Pufferlösung vom pH-Wert 7 gewaschen. Die erhaltenen sauberen Kutikel wurden schließlich auf Teflonplättchen aufgezogen und mit einem schwachen Luftstrahl geglättet und getrocknet.

Im nächsten Schritt wurden die so gewonnenen Kutikelmembranen für Membran-Transport-Untersuchungen in Diffusionszellen (= Transportkammern) aus Edelstahl eingelegt. Dazu wurden die Kutikeln mit einer Pinzette mittig auf die mit Silikonfett bestrichenen Ränder der Diffusionszellen plaziert und mit einem ebenfalls gefetteten Ring verschlossen. Die Anordnung war so gewählt worden, dass die morphologische Außenseite der Kutikeln nach außen, also zur Luft, gerichtet war, während die ursprüngliche Innenseite dem Inneren der Diffusionszelle zugewandt war. Die Diffusionszellen waren mit Wasser bzw. mit einem Gemisch aus Wasser und Lösungsmittel gefüllt.

Zur Bestimmung der Penetration wurden jeweils 9 µl einer Spritzbrühe der nachstehend genannten Zusammensetzung auf die Außenseite einer Kutikula appliziert.

### Spritzbrühe A

| | |
|---|---|
| 0,2 g | Thiacloprid |
| 0,4 g | Sonnenblumenöl |
| 0,4 g | Formulierhilfsstoffe |
| | in 1 Liter Wasser. |

### Spritzbrühe B

| | |
|---|---|
| 0,2 g | Thiacloprid |
| 0,5 g | 2-Ethyl-hexyl-alkoxylat der Formel (Ic-2) |
| 0,3 g | Formulierhilfsstoffe |
| | in 1 Liter Wasser. |

### Spritzbrühe C

| | |
|---|---|
| 0,2 g | Thiacloprid |
| 0,4 g | Sonnenblumenöl |
| 0,2 g | 2-Ethyl-hexyl-alkoxylat der Formel (Ic-2) |
| 0,2 g | Formulierhilfsstoffe |
| | in 1 Liter Wasser. |

### Spritzbrühe D

| | |
|---|---|
| 0,2 g | Thiacloprid |
| 0,3 g | Formulierhilfsstoffe |
| | in 1 Liter Wasser, |

(hergestellt aus handelsüblichem Suspensionskonzentrat durch Verdünnen mit Wasser).

In den Spritzbrühen wurde jeweils CIPAC-Wasser verwendet.

Nach dem Auftragen der Spritzbrühen ließ man jeweils das Wasser verdunsten, drehte dann jeweils die Kammern um und stellte sie in thermostatisierte Wannen, wobei sich unter der Außenseite der Kutikula jeweils eine gesättigte wässrige Calciumnitrat-4-hydrat-Lösung befand. Die einsetzende Penetration fand daher bei einer relativen Luftfeuchtigkeit von 56 % und einer eingestellten Temperatur von 25°C statt. In regelmäßigen Abständen wurden mit einer Spritze Proben entnommen und mittels HPLC auf den Gehalt an penetriertem Wirkstoff hin untersucht.

Die Versuchsergebnisse gehen aus der folgenden Tabelle hervor. Bei den angegebenen Zahlen handelt es sich um Durchschnittswerte von 8 Messungen.

**Tabelle II**

| | Wirkstoffpenetration in % nach | | |
|---|---|---|---|
| | 5h | 10h | 20h |
| A | 1 | 3 | 4 |
| B | 10 | 16 | 20 |
| C | 6 | 17 | 40 |
| D | | | 1 |

## Patentansprüche

1. Suspensionskonzentrate aufÖlbasis, bestehend aus
- mindestens einem bei Raumtemperatur festen agrochemischen Wirkstoff,
- mindestens einem Penetrationsförderer, ausgewählt aus Alkanol-alkoxylaten der Formel
R-O-(-AO)ₘH (I)
in welcher
R für geradkettiges oder verzweigtes Alkyl mit 4 bis 20 Kohlenstoffatomen steht,
AO für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
m für Zahlen von 2 bis 30 steht,
- mindestens einem Pflanzenöl,
- mindestens einem nicht-ionischen Tensid bzw. Dispergierhilfsmittel und/oder mindestens einem anionischen Tensid bzw. Dispergierhilfsmittel und
- gegebenenfalls einem oder mehreren Zusatzstoffen aus den Gruppen der Emulgiermittel, der schaumhemmenden Mittel, der Konservierungsmittel, der Antioxydantien und/oder der Farbstoffe.

2. Suspensionskonzentrate gemäß Anspruch 1, **dadurch gekennzeichnet, dass** als agrochemischer Wirkstoff ein Fungizid, Bakterizid, Insektizid, Akarizid, Nematizid, Moluskizid, Herbizid, Pflanzenwuchsregulator, Pflanzennährstoff und/oder ein Repellent enthalten ist.

3. Suspensionskonzentrate gemäß Anspruch 1, **dadurch gekennzeichnet, dass** als agrochemischer Wirkstoff Thiacloprid enthalten ist.

4. Suspensionskonzentrate gemäß Anspruch 1, **dadurch gekennzeichnet, dass** als agrochemische Wirkstoffe Thiacloprid und β-Cyfluthrin enthalten sind.

5. Suspensionskonzentrate gemäß Anspruch 1, **dadurch gekennzeichnet, dass** als Alkanol-Alkoxylat 2-Ethyl-hexyl-alkoxylat der Formel 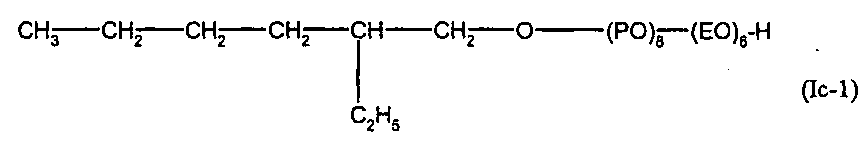 in welcher
EO für -CH₂-CH₂-O- steht,
PO für 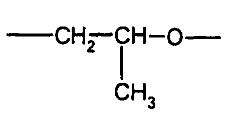 steht und
die Zahlen 8 und 6 Durchschnittswerte darstellen,
enthalten ist.

6. Suspensionskonzentrate gemäß Anspruch 1, **dadurch gekennzeichnet, dass** als Alkanol-alkoxylat 2-Ethyl-hexyl-alkoxylat der Formel 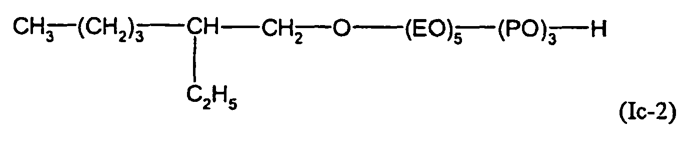 in welcher
EO für -CH₂-CH₂-O- seht,
PO für 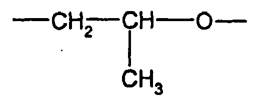 steht und
die Zahlen 5 und 3 Durchschnittswerte darstellen,
enthalten ist.

7. Suspensionskonzentrate gemäß Anspruch 1, **dadurch gekennzeichnet, dass** als Pflanzenöl Sonnenblumenöl, Rapsöl, Olivenöl, Rizinusöl, Rüböl, Maiskemöl, Baumwollsaatöl und/oder Sojabohnenöl enthalten ist.

8. Suspensionskonzentrate gemäß Anspruch 1, **dadurch gekennzeichnet, dass** der Gehalt
- an agrochemischen Wirkstoffen zwischen 5 und 30 Gew.-%,
- an Alkanol-Alkoxylat der Formel (I) zwischen 5 und 55 Gew.-%,
- an Pflanzenöl zwischen 15 und 55 Gew.-%,
- an Tensiden bzw. Dispergierhilfsmitteln zwischen 2,5 und 30 Gew.-% und
- an Zusatzstoffen zwischen 0 und 25 Gew.-%
liegt.

9. Verfahren zur Herstellung von Suspensionskonzentraten gemäß Anspruch 1, **dadurch gekennzeichnet, dass** man
- mindestens einen bei Raumtemperatur festen agrochemischen Wirkstoff,
- mindestens einen Penetrationsförderer, ausgewählt aus Alkanol-alkoxylaten der Formel
R-O-(-AO)ₘH (I)
in welcher
R für geradkettiges oder verzweigtes Alkyl mit 4 bis 20 Kohlenstoffatomen steht,
AO für einen Ethylenoxid-Rest, einen Propylenoxid-Rest, einen Butylenoxid-Rest oder für Gemische aus Ethylenoxid- und Propylenoxid-Resten oder Butylenoxid-Resten steht und
m für Zahlen von 2 bis 30 steht,
- mindestens ein Pflanzenöl,
- mindestens ein nicht-ionisches Tensid bzw. Dispergierhilfsmittel und/oder mindestens ein anionisches Tensid bzw. Dispergierhilfsmittel und
- gegebenenfalls einen oder mehrere Zusatzstoffe aus den Gruppen der Emulgiermittel, der schaumhemmenden Mittel, der Konservierungsmittel, der Antioxydantien und/oder der Farbstoffe.
miteinander vermischt und die entstehende Suspension gegebenenfalls anschließend mahlt.

10. Verwendung von Suspensionskonzentraten gemäß Anspruch 1 zur Applikation der enthaltenen agrochemischen Wirkstoffe auf Pflanzen und/oder deren Lebensraum.

## Claims

1. Oil-based suspension concentrates, consisting of
- at least one agrochemical active compound which is solid at room temperature,
- at least one penetration promoter selected from alkanol alkoxylates of the formula
R-O-(-AO)ₘH (I)
in which
R represents straight-chain or branched alkyl having 4 to 20 carbon atoms,
AO represents an ethylene oxide radical, a propylene oxide radical, a butylene oxide radical or mixtures of ethylene oxide and propylene oxide radicals or butylene oxide radicals and
m represents numbers from 2 to 30,
- at least one vegetable oil,
- at least one non-ionic surfactant or dispersing aid and/or at least one anionic surfactant or dispersing aid and
- optionally one or more additives from the groups consisting of the emulsifying agents, the antifoam agents, the preservatives, the antioxidants and/or the colourants.

2. Suspension concentrates according to Claim 1, **characterized in that** the agrochemical active compound contained is a fungicide, bactericide, insecticide, acaricide, nematicide, molluscicide, herbicide, plant growth regulator, plant nutrient and/or a repellent.

3. Suspension concentrates according to Claim 1, **characterized in that** the agrochemical active compound contained is thiacloprid.

4. Suspension concentrates according to Claim 1, **characterized in that** the agrochemical active compounds contained are thiacloprid and β-cylluthrin.

5. Suspension concentrates according to Claim 1, **characterized in that** the alkanol alkoxylate contained is 2-ethyl-hexyl alkoxylate of the formula 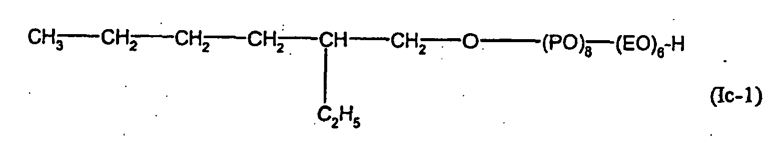 in which
EO represents -CH₂-CH₂-O-,
PO represents 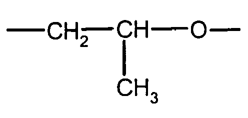 and
the numbers 8 and 6 are average values.

6. Suspension concentrates according to Claim 1, **characterized in that** the alkanol alkoxylate contained is 2-ethyl-hexyl alkoxylate of the formula 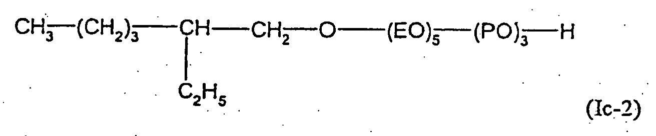 in which
EO represents -CH₂-CH₂-O-,
PO represents 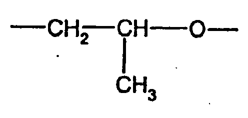 and
the numbers 5 and 3 are average values.

7. Suspension concentrates according to Claim 1, **characterized in that** the vegetable oil contained is sunflower oil, rapeseed oil, olive oil, castor oil, colza oil, maize germ oil, cottonseed oil and/or soya bean oil.

8. Suspension concentrates according to Claim 1, **characterized in that** the content
- of agrochemical active compounds is between 5 and 30 % by weight,
- of alkanol alkoxylate of the formula (I) is between 5 and 55 % by weight,
- of vegetable oil is between 15 and 55 % by weight,
- of surfactants or dispersing aids is between 2.5 and 30 % by weight and
- of additives is between 0 and 25 % by weight.

9. Process for the preparation of suspension concentrates according to Claim 1, **characterized in that**
- at least one agrochemical active compound which is solid at room temperature,
- at least one penetration promoter selected from alkanol alkoxylates of the formula
R-O-(-AO)ₘH (I)
in which
R represents straight-chain or branched alkyl having 4 to 20 carbon atoms,
AO represents an ethylene oxide radical, a propylene oxide radical, a butylene oxide radical or mixtures of ethylene oxide and propylene oxide radicals or butylene oxide radicals and
m represents numbers from 2 to 30,
- at least one vegetable oil,
- at least one non-ionic surfactant or dispersing aid and/or at least one anionic surfactant or dispersing aid and
- optionally one or more additives from the groups consisting of the emulsifying agents, the antifoam agents, the preservatives, the antioxidants and/or the colourants
are mixed with one another and the resulting suspension is optionally subsequently ground.

10. Use of suspension concentrates according to Claim 1 for the application of the agrochemical active compounds contained to plants and/or their habitat.

## Revendications

1. Concentrés en suspension à base d'huile, constitués
- d'au moins une substance active agrochimique solide à la température ambiante,
- d'au moins une substance facilitant la pénétration, choisie entre des alkoxylates d'alcanols de formule
R-O-(-AO)ₘH (I)
dans laquelle
R désigne un reste alkyle linéaire ou ramifié ayant 4 à 20 atomes de carbone,
AO désigne un reste oxyde d'éthylène, un reste oxyde de propylène, un reste oxyde de butylène ou des mélanges de restes oxyde d'éthylène et oxyde de propylène ou oxyde de butylène, et
m représente des nombres de 2 à 30,
- d'au moins une huile végétale,
- d'au moins un agent tensioactif ou auxiliaire de dispersion non ionique et/ou d'au moins un agent tensioactif ou auxiliaire de dispersion anionique, et
- le cas échéant, d'un ou plusieurs additifs choisis dans les groupes des émulsifiants, des agents inhibiteurs de mousse, des conservateurs, des antioxydants et/ou des colorants.

2. Concentrés en suspension suivant la revendication 1, **caractérisés en ce qu'**ils contiennent comme substance active agrochimique un fongicide, un bactéricide, un insecticide, un acaricide, un nématicide, un mollusquicide, un herbicide, un régulateur de croissance des plantes, une substance nutritive pour les plantes et/ou un répulsif.

3. Concentrés en suspension suivant la revendication 1, **caractérisés en ce qu'**ils contiennent du thiaclopride comme substance active agrochimique.

4. Concentrés en suspension suivant la revendication 1, **caractérisés en ce qu'**ils contiennent du thiaclopride et de la β-cyfluthrine comme substances actives agrochimiques.

5. Concentrés en suspension suivant la revendication 1, **caractérisés en ce qu'**ils contiennent comme alkoxyalcoolate d'alcanol un alkoxylate de 2-éthyl-hexyle de formule 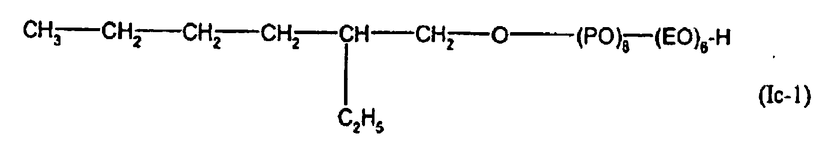 dans laquelle
EO représente -CH₂-CH₂-O-,
PO représente 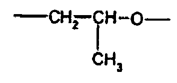 et
les nombres 8 et 6 représentent des valeurs moyennes.

6. Concentrés en suspension suivant la revendication 1, **caractérisés en ce qu'**ils contiennent comme alkoxyalcoolate d'alcanol un alkoxylate de 2-éthyl-hexyle de formule 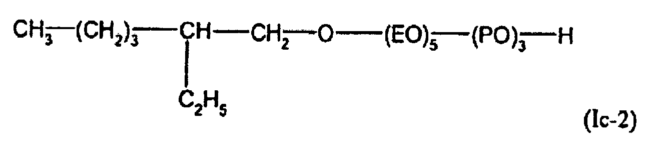 dans laquelle
EO représente -CH₂-CH₂-O-,
PO représente 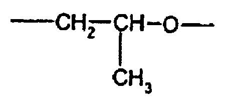 et
les nombres 5 et 3 représentent des valeurs moyennes.

7. Concentrés en suspension suivant la revendication 1, **caractérisés en ce qu'**ils contiennent comme huile végétale de l'huile de tournesol, de l'huile de colza, de l'huile d'olive, de l'huile de ricin, de l'huile de navette, de l'huile de germes de maïs, de l'huile de graines de cotonnier et/ou de l'huile de soja.

8. Concentrés en suspension suivant la revendication 1, **caractérisés par**
- une teneur en substances actives agrochimiques comprise entre 5 et 30 % en poids,
- une teneur en alkoxylate d'alcanol de formule (I) comprise entre 5 et 55 % en poids,
- une teneur en huile végétale comprise entre 15 et 55 % en poids,
- une teneur en agents tensioactifs ou en auxiliaires de dispersion comprise entre 2,5 et 30 % en poids et
- une teneur en additifs comprise entre 0 et 25 % en poids.

9. Procédé de production de concentrés en suspension suivant la revendication 1, **caractérisé en ce qu'**on mélange
- au moins une substance active agrochimique solide à la température ambiante avec
- au moins une substance facilitant la pénétration, choisie entre des alkoxylates d'alcanols de formule
R-O-(-AO)ₘH (I)
dans laquelle
R désigne un reste alkyle linéaire ou ramifié ayant 4 à 20 atomes de carbone,
AO désigne un reste oxyde d'éthylène, un reste oxyde de propylène, un reste oxyde de butylène ou des mélanges de restes oxyde d'éthylène et oxyde de propylène ou oxyde de butylène, et
m représente des nombres de 2 à 30,
- au moins une huile végétale,
- au moins un agent tensioactif ou auxiliaire de dispersion non ionique et/ou au moins un agent tensioactif ou auxiliaire de dispersion anionique, et
- le cas échéant, un ou plusieurs additifs choisis dans les groupes des émulsifiants, des inhibiteurs de mousse, des conservateurs, des antioxydants et/ou des colorants,
et on broie ensuite le cas échéant la suspension produite.

10. Utilisation de concentrés en suspension suivant la revendication 1 pour l'application des substances actives agrochimiques qu'ils contiennent à des plantes et/ou à leur milieu.
